# EUROPEAN PATENT APPLICATION

(11) **EP 3 903 742 A1**
(43) Date of publication of application: **03.11.2021**
(21) Application number: 19903125.3
(22) Date of filing: 24.12.2019
(51) Int. Cl.: A61F 2/95, A61M 25/092

(54) **ADJUSTABLE CURVED SHEATH TUBE AND STENT DELIVERY DEVICE**

(30) Priority: 28.12.2018 CN 201811625262; 28.12.2018 CN 201822240012 U
(71) Applicant: Hangzhou Endonom Medtech Co., Ltd, Hangzhou, Zhejiang 310051 (CN)
(72) Inventor: WANG, Yongsheng, Hangzhou, Zhejiang 310051 (CN); LI, Jianmin, Hangzhou, Zhejiang 310051 (CN)
(74) Representative: Krauns, Christian
(86) International application number: PCT/CN2019/128097
(87) International publication number: WO 2020/135456

(57) **Abstract**

An adjustable bendable sheath (4), which is configured to deliver a stent (200) into a vessel, includes a bendable tube (41) and a pulling wire (42). One end of the pulling wire (42) is fixedly connected to one end of the bendable tube (41), the other end of the pulling wire (42) extends out of the bendable tube (41) and then into the bendable tube (41), and at the last the pulling wire extends out of the other end of the bendable tube (41). When the other end of the pulling wire (42) is pulled, the pulling wire (42) drives one end of the bendable tube (41) to move close to the other end of the pulling wire (42), so as to bend the bendable tube (41). The present application further relates to a stent delivery device (100) having an adjustable bendable sheath (4), which is capable of effectively releasing a stent (200) at a lesion site of the vessel.

## Description

### TECHNICAL FIELD

The present application relates to the technical field of medical instruments, and more particularly to an adjustable bendable sheath and a stent delivery device.

### BACKGROUND

Aortic diseases, such as aortic aneurysm and aortic dissection, are one kind of the most pernicious and hardest vascular surgical diseases to treat. Traditional treatment methods, such as laparotomy and prosthetic vessel replacement, have the risks of severe surgical trauma and high fatality rate. In recent years, a minimally invasive and simple interventional operation method has been developed, in which a covered stent is implanted in a lesion site of a vessel, and the covered stent conforms to the inner wall of the vessel to isolate the lesion site of the vessel from the blood flow. The covered stent can not only allow the blood to flow through normally, but also protect the lesion site of the vessel and effectively repair the lesion site of the vessel. However, how to provide an adjustable bendable sheath and a stent delivery device capable of effectively releasing the stent into the lesion site of the vessel (including a curved vessel) to improve the effect of treating vascular diseases has become a technical problem to be solved.

### SUMMARY

The present application provides a stent delivery device capable of effectively releasing a stent into a lesion site of a vessel.

In one aspect, the present application provides an adjustable bendable sheath for a stent delivery device. The adjustable bendable sheath includes a bendable tube; and a pulling wire, wherein one end of the pulling wire is fixedly connected to one end of the bendable tube, while the other end of the pulling wire extends out of the bendable tube and into the bendable tube until the pulling wire extends out of the other end of the bendable tube. When the other end of the pulling wire is pulled, the length of the one end of the pulling wire, which is positioned outside the bendable tube, is reduced, and the bendable tube is bent under the action of the pulling wire.

In another aspect, the present application provides a stent delivery device configured to release a stent into the vessel. The stent delivery device includes the adjustable bendable sheath and a sheathed tube adjusting assembly, wherein the sheath adjusting assembly is connected to the other end of the pulling wire, and the sheath adjusting assembly is configured to pull the pulling wire.

By fixing one end of the pulling wire on the bendable tube, when the other end of the pulling wire is pulled, a fixed portion moves towards the other end of the pulling wire. During the process, the bendable tube is bent. One part of the pulling wire is arranged outside the bendable tube, and the other part of the pulling wire is arranged within the bendable tube, so that the pulling wire is capable of extending along a curvature of the bendable tube, and thus the pulling wire exposed outside the bendable tube is prevented from scratching the wall of the vessel or other structures. Accordingly, the using experience of the adjustable bendable sheath is improved. Moreover, the bending degree of the adjustable bendable sheath is adjusted by adjusting the length the pulling wire is shortened, so that the adjustable bendable sheath is capable of entering into vessels with different curvatures.

### BRIEF DESCRIPTION OF THE DRAWINGS

In order to illustrate the technical solutions of the embodiments according to the present application more clearly, drawings used in the description of the embodiments according to the present application will be briefly introduced below. It should be appreciated that the drawings described below merely illustrate some embodiments of the present application, and other drawings may be obtained by those skilled in the art without departing from the scope of the drawings.
FIG. 1 is a schematic view showing a structure of a stent delivery device according to an embodiment of the present application.
FIG. 2 is a cross-sectional view showing a stent delivery device according to an embodiment of the present application.
FIG. 3 is a schematic partial enlarged view of an area A in FIG. 2;
FIG. 4 is a cross-sectional view showing an adjustable bendable sheath according to an embodiment of the present application;
FIG. 5 is a cross-sectional view showing a second adjustable bendable sheath according to an embodiment of the present application;
FIG. 6 is a partial cross-sectional view showing a third adjustable bendable sheath according to an embodiment of the present application;
FIG. 7 is a partial cross-sectional view showing a fourth adjustable bendable sheath according to an embodiment of the present application;
FIG. 8 is a cross-sectional view showing a sheath adjusting assembly of a stent delivery device according to an embodiment of the present application;
FIG. 9 is a partial perspective view showing a proximal end of a stent delivery device according to an embodiment of the present application;
FIG. 10 is a cross-sectional view showing a sheath sliding assembly of a stent delivery device according to an embodiment of the present application;
FIG. 11 is a partial enlarged cross-sectional view showing a switch of a sheath sliding assembly of a stent delivery device according to an embodiment of the present application;
FIG. 12 is a partial cross-sectional view showing a distal end of a stent delivery device according to an embodiment of the present application; and
FIG. 13 is an exploded view showing a releasing assembly of a stent delivery device according to an embodiment of the present application.

### DESCRIPTION OF THE EMBODIMENTS

The technical solutions of the embodiments of the present application will be clearly and fully described below in combination with accompanying drawings in the embodiments of the present application. For ease of description, "proximal end", "distal end" and "first direction" are involved, wherein the term "proximal end" refers to one end away from an operating end (a handle end) of the stent delivery device, and the term "distal end" refers to one end close to the operating end of the stent delivery device, and the term "first direction" refers to an extending direction of the adjustable bendable sheath and the stent delivery device.

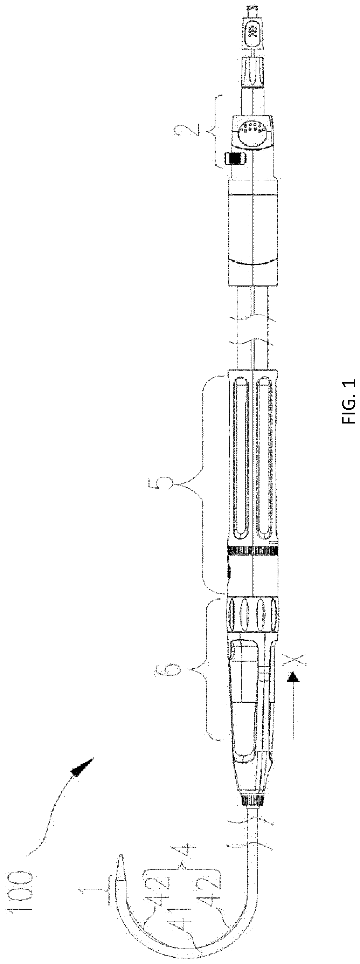

Referring to FIG. 1 to FIG. 3, FIG. 1 shows a stent delivery device 100 according to an embodiment of the present application. The stent delivery device 100 is configured to release the stent 200 into a lesion site of a vessel. The stent 200 includes a tubular rigid wire frame and a polymer membrane fixed on an outer peripheral surface of the tubular rigid wire frame. The tubular rigid wire frame may be obtained by forming an elastic rigid wire bended in a Z-shaped manner into a plurality of rings, and then the stent 200 with a membrane is formed by stitching or bonding the plurality of rings with the polymer membrane. During use, the stent 200 is loaded into the stent delivery device 100 after being compressed, and delivered to the lesion site of the vessel by the stent delivery device 100 and then released. Due to the elasticity of the tubular rigid wire frame, the stent 200 automatically returns toa straight tube and closely conforms to an inner wall of the vessel to isolate the lesion site of the vessel from the blood flow, thereby achieving the treatment.

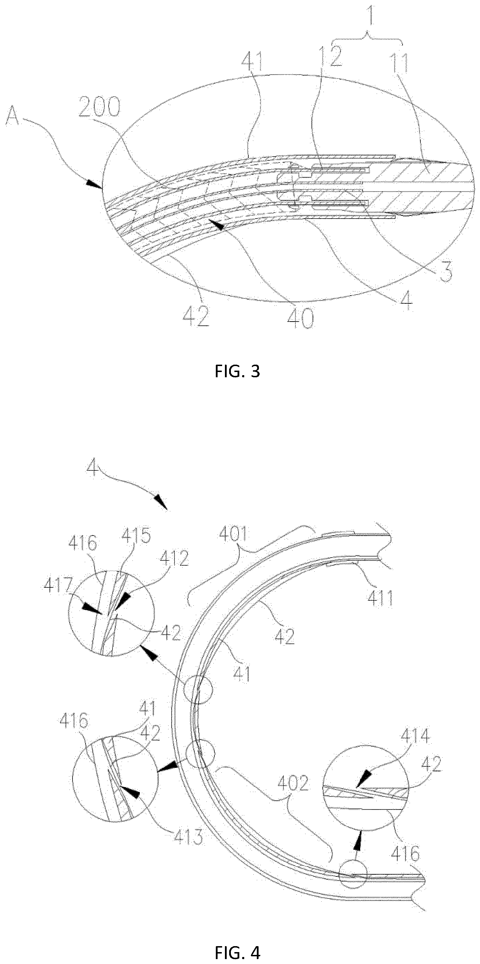

Referring to FIG. 4, FIG. 4 shows an adjustable bendable sheath 4 according to an embodiment of the present application, which is applied in a stent delivery device 100. The adjustable bendable sheath 4 may be used to carry the stent 200 and deliver the stent 200 into the lesion site of the vessel. The adjustable bendable sheath 4 includes a bendable tube 41 and a pulling wire 42. One end of the pulling wire 42 is fixedly connected to one end of the bendable tube 41 (a proximal end of the bendable tube 41). The other end of the pulling wire 42 (a distal end of the pulling wire 42) passes out of the bendable tube 41, then enters back into the bendable tube 42, and at the last the other end of the pulling wire extends out of the other end of the bendable tube 42 (the distal end of the bendable tube 41). In other words, a part of the pulling wire 42 is arranged outside an outer tube wall of the bendable tube 41. Another part of the pulling wire 42 is arranged within the bendable tube 41. When the other end of the pulling wire 42 (a distal end of the pulling wire 42) is pulled, the pulling wire 42 drives one end of the bendable tube 41 to approach to the other end of the pulling wire 42 (the distal end of the pulling wire 42) to bend the bendable tube 41.

It should be understood that when the pulling wire 42 is arranged within the bendable tube 41, the extending direction of the pulling wire 42 is consistent with that of the bendable tube 41. When the pulling wire 42 is arranged outside the bendable tube 41, the pulling wire 42 extends close to the outer tube wall of the bendable tube 41.

It should be understood that when the other end of the pulling wire 42 is pulled, the length of the pulling wire 42 positioned outside the bendable tube 41 is reduced, and the bendable tube 41 is bent under the action of the pulling wire 42.

By fixing one end of the pulling wire 42 on the bendable tube 41, when the other end of the pulling wire 42 is pulled, the fixing portion moves towards the other end of the pulling wire 42, during this process, the bendable tube 41 is bent. One part of the pulling wire 42 is arranged outside the bendable tube 41, and the other part of the pulling wire 42 is arranged within the bendable tube 41, so that the pulling wire 42 can extend along a curvature of the bendable tube 41, and thus the pulling wire 42 exposed outside the bendable tube 41 is prevented from scratching the wall of the vessel or other structures. Accordingly, the using experience of the adjustable bendable sheath 4 is improved. Moreover, the bending degree of the adjustable bendable sheath 4 is adjusted by means of adjusting the length the pulling wire 42 is shortened, to allow the adjustable bendable sheath 4 to extend into vessels of different curvatures.

In a first possible implementation, with reference to FIG. 4, an outer tube wall of the bendable tube 41 is provided with a fixed portion 411 and a first opening 412. The one end of the pulling wire 42 is fixed to the fixed portion 411. The other end of the pulling wire 42 extends into the bendable tube 41 via the first opening 412. That is, the pulling wire 42 is exposed between the fixed portion 411 and the first opening 412.

Particularly, the fixed portion 411 may be a fixed ring. The one end of the pulling wire 42 is fixed between the fixed portion 411 and an outer peripheral wall of the bendable tube 41. The fixed portion 411 is tightly mounted around the outer periphery of the bendable tube 41, so that one end of the pulling wire 42 is fixed on the fixed portion 411 of the bendable tube 41. Certainly, in other implementations, the fixed portion 411 may be a post. The one end of the pulling wire 42 may be tied to the fixed portion 411, or the pulling wire 42 extends through the fixed portion 411 and a big end of the pulling wire 42 abuts against the fixed portion 411, or the pulling wire 42 may be fixed with the fixed portion 411 by means of welding, bonding, snap-fitting, and the like, or the one end of the pulling wire 42 may be integrally formed with the fixed portion 411 as one piece.

When the pulling wire 42 is pulled, the pulling wire 42 between the fixed portion 411 and the first opening 412 is shortened, and the pulling wire 42 exerts pressing forces at the fixed portion 411 and the first opening 412 of the bendable tube 41, respectively. The fixed portion 411 and the first opening 412 of the bendable tube 41 are approaching to each other under the two pressing forces, so that the bendable tube 41 between the fixed portion 411 and the first opening 412 is bent, and in turn the adjustable bendable sheath 4 is bent.

Further, with reference to FIG. 4, the outer tube wall of the bendable tube 41 is further provided with a second opening 413. The first opening 412 is positioned between the second opening 413 and the fixed portion 411. The other end of the pulling wire 42 extends out of the outer tube wall of the bendable tube 41 via the second opening 413.

It should be understood that, with reference to FIG. 4, the second opening 413 and the first opening 412 are spaced apart in an axial direction of the bendable tube 41. Particularly, when the pulling wire 42 is pulled, the pulling wire 42 between the first opening 412 and the second opening 413 tightly conform to the bendable tube 41, so that the bendable tube 41 forms a supporting section of the pulling wire 42. In this way, an action force of the pulling wire 42 may be evenly distributed on an inner wall between the first opening 412 and the second opening 413, which prevents the surface of the bendable tube 41 from being broken due to an excessive local action force of the bendable tube 41. As a result, the reliability and the service life of the adjustable bendable sheath 4 are improved.

Further, with reference to FIG. 4, the first opening 412 and the second opening 413 are collinear along an axial direction of the bendable tube 41.

Particularly, the second opening 413 is collinear with the first opening 412 in the axial direction of the bendable tube 41, so that the adjustable bendable sheath 4 is bent within a plane, that is, the bendable sheath 4 is bent in a hook shape along an arc.

Further, with reference to FIG. 4, the outer tube wall of the bendable tube 41 is further provided with a third opening 414. The second opening 413 is positioned between the third opening 414 and the first opening 412. The other end of the pulling wire 42 extends into the bendable tube 41 via the third opening 414.

In this implementation, two sections of the pulling wire 42 are arranged outside the bendable tube 41. When the pulling wire 42 is pulled, the pulling wire 42 exposed outside the bendable tube 41 is shortened, which drives the bendable tube 41 between the fixed portion 411 and the first opening 412 and the bendable tube 41 between the second opening 413 and the third opening 414 to be bent. Two areas on the bendable tube 41 are bent, which can increase the bending degree and the bending speed of the bendable sheath 4, so that when the pulling wire 42 is shortened, the bendable tube 41 is capable of responding quickly and presenting a certain bending degree.

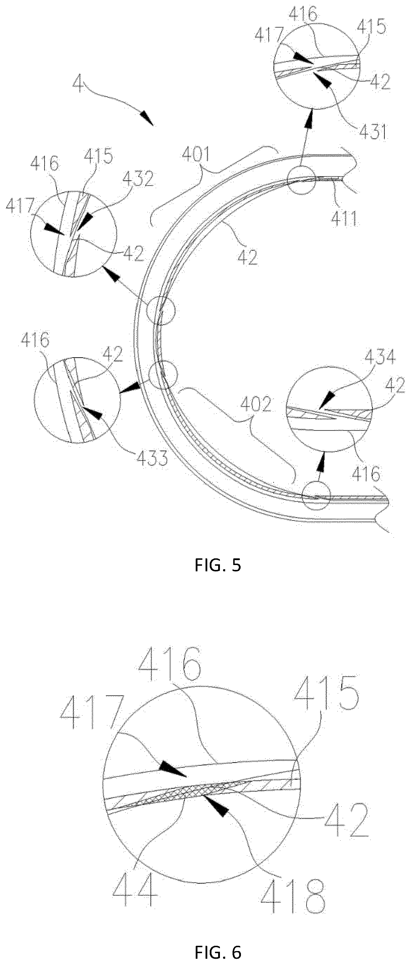

With reference to FIG. 4, when the bendable tube 41 is bent in an arc shape, the first bending area 401 of the bendable tube 41 is bent along a bottom-left direction from the fixed portion 411 (as shown in FIG. 5), and the second bending area 402 of the bendable tube 41 is bent along a bottom-right direction from the second opening 413 (as shown in FIG. 5). The bendable tube 41 between the first opening 412 and the second opening 413 forms an inflection point of the bendable tube 41. The size and the shape of the hook formed by bending the flexible tube 41 may be adjusted by means of adjusting positions where the first opening 412 and the second opening 413 are located and a distance between the first opening 412 and the second opening 413, so as to adapt to vessels with different thicknesses and vessels with different curvatures. Accordingly, the application range of the adjustable bendable sheath 4 is increased.

Further, with reference to FIG. 6, the first opening 412, the second opening 413 and the third opening 414 are arranged sequentially along the axial direction of the bendable tube 41. Preferably, the first opening 412, the second opening 413 and the third opening 414 are collinear along the axial direction of the bendable tube 41, so that the bendable tube 41 retains bending within one plane. Particularly, the bendable tube 41 may be bent in a hook shape.

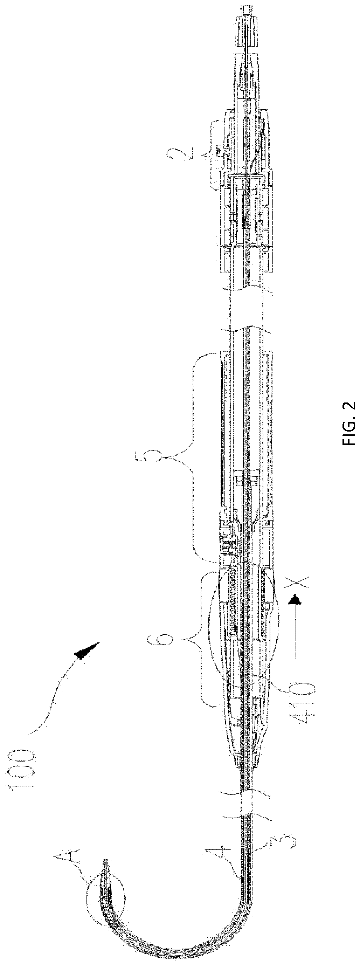

In a possible implementation, with reference to FIG. 2, the outer tube wall of the bendable tube 41 is further provided with a fourth opening 410. The fourth opening 410 is close to the other end of the bendable tube 41, and the other end of the pulling wire 42 extends out of the bendable tube 41 via the fourth opening 410.

In another possible implementation, which is different from the previous implementation in that, the fourth opening 410 is provided in an end surface of the other end of the bendable tube 41. The other end of the pulling wire 41 extends out of the bendable tube 41 via the fourth opening 410.

In combination with the foregoing implementation, with reference to FIG. 4, the bendable tube 41 is provided with an inner lining layer 416 and an outer lining layer 415 mounted around the inner lining layer 416, wherein a gap is provided between the outer lining layer 415 and the inner lining layer 416 to form an inner cavity. The first opening 412, the second opening 413, the third opening 414 and the fourth opening 410 are provided in the outer lining layer 415 and are communicating with the inner cavity 417. The pulling wire 42 is partially arranged in the inner cavity 417.

It should be understood that as the pulling wire 42 is positioned in the inner cavity 417 of the bendable tube 41, the inner lining layer 416 separates the pulling wire 42 from other structures. When the pulling wire 42 is pulled, the pulling wire 42 will not enwind other structures. In this way, the bending efficiency of the bendable tube 41 may be increased. Furthermore, the inner cavity 417 extends along the axial direction of the bendable tube 41, so that the pulling wire 42 extends along the axial direction of the bendable tube 41.

It should be understood that the bendable tube 41 has a pre-bending angle in an initial state, and the pre-bending angle may be ranged from 5° to 90°. The pre-bending angle is set to enable the bendable tube 41 to be bent along a pre-bending direction when being driven by the pulling wire 42, so that the adjustable bendable sheath 4 may be quickly bent along a preset bending direction. Therefore, the efficiency of the bending operation is increased.

It should be understood that the maximum bending angle of the bendable tube 41 may be ranged from 120° to 200°, so that the adjustable bendable sheath body 4 is capable of entering into a vessel with a greater curvature, so as to increase the application range of the stent 200 delivery device 100.

Particularly, the material of the pulling wire 42 may have a certain structural strength and is bendable. The pulling wire 42 may be a stainless-steel wire, a memory alloy wire, a polymer material wire, or the like. 1 to 5 pulling wires 42 may be provided which are arranged in parallel.

In another possible implementation, with reference to FIG. 5, a fixed portion 411 is provided within the bendable tube 41. One end of the pulling wire 42 is fixed to the fixed portion 411. The outer tube wall of the bendable tube 41 is provided with a first through hole 431 and a second through hole 432. The first through hole 431 is positioned between the second through hole 432 and the fixed portion 411. The pulling wire 42 extends out of the bendable tube 41 via the first through hole 431 and into the bendable tube 41 via the second through hole 432.

In this implementation, the structure of the fixed portion 411 may refer to the fixed portion 411 of the first implementation, which will not be repeated again herein.

With reference to FIG. 5, when the pulling wire 42 is pulled, the pulling wire 42 between the first through hole 431 and the second through hole 432 is shortened, the pulling wire 42 exerts pressing forces at the first through hole 431 and the second through hole 431 of the flexible tube 41, respectively. The first through hole 431 and the second through hole 432 of the bendable tube 41 are approaching to each other under the two pressing forces, so that the bendable tube 41 between the first through hole 431 and the second through hole 432 is bent, and in turn the adjustable bendable sheath 4 is bent.

Further, with reference to FIG. 5, the outer tube wall of the bendable tube 41 is further provided with a third through hole 433. The second through hole 432 is positioned between the third through hole 433 and the first through hole 431. The pulling wire 42 extends out of the bendable tube 41 via the third through hole 433. It should be understood that the pulling wire 42 extends out of the outer tube wall of the bendable tube 41 via the third through hole 433.

By providing the third through hole 433, when the pulling wire 42 is pulled, the pulling wire 42 between the fixed portion 411 and the first through hole 431 and the pulling wire 42 between the second through hole 432 and the third through hole 433 tightly conform to the bendable tube 41, so that the bendable tube 41 forms a supporting section of the pulling wire 42. In this way, an action force of the pulling wire 42 may be evenly distributed on the inner wall between the fixed portion 411 and the first through hole 431 and the inner wall between the second through hole 432 and the third through hole 433, which prevents the surface of the bendable tube 41 from being broken due to excessive action force of the bendable tube 41. As a result, the reliability and the service life of the adjustable bendable sheath 4 are improved.

Further, with reference to FIG. 5, the outer tube wall of the bendable tube 41 is further provided with a fourth through hole 434. The fourth through hole 434 is provided at one side of the third through hole 433 which is away from the second through hole 432. The pulling wire 42 extends into the bendable tube 41 via the fourth through hole 434.

In this implementation, two sections of the pulling wire 42 are arranged outside the bendable tube 41. When the pulling wire 42 is pulled, the pulling wire 42 exposed outside the bendable tube 41 is shortened, which drives the bendable tube 41 between the first through hole 431 and the second through hole 432 and the bendable tube 41 between the third through hole 433 and the fourth through hole 434 to be bent. Two areas on the bendable tube 41 can be bent, which can increase the bending degree and the bending speed of the bendable sheath 4, so that when the pulling wire 42 is shortened, the bendable tube 41 is capable of responding quickly and presenting a certain bending degree.

Further, with reference to FIG. 5, the first through hole 431, the second through hole 432, the third through hole 433 and the fourth through hole 434 are arranged sequentially along the axial direction of the bendable tube 41. Preferably, the first through hole 431, the second through hole 432, the third through hole 433 and the fourth through hole 434 are collinear along the axial direction of the bendable tube 41, so that the bendable tube 41 retains bending within one plane. Particularly, the bendable tube 41 may be bent in a hook shape.

Particularly, with reference to FIG. 5, the bendable tube 41 is provided with an inner lining layer 416, an outer lining layer 415 mounted around the inner lining layer 416, and an inner cavity 417 defined between the outer lining layer 415 and the inner lining layer 416. The first through hole 431, the second through hole 432, the third through hole 433 and the fourth through hole 434 are provided in the outer lining layer 415 and are communicating with the inner cavity 417. The pulling wire 42 partially extends along the inner cavity 417.

It should be understood that the pulling wire 42 is arranged within the inner cavity 417, the inner lining layer 416 isolates the pulling wire 42 from other structures. When the pulling wire 42 is pulled, the pulling wire 42 does not interfere with other structures. In this way, the bending efficiency of the bendable tube 41 may be increased. Furthermore, the inner cavity 417 extends along the axial direction of the bendable tube 41 to guide the pulling wire 42 to extend along the axial direction of the bendable tube 41.

In combination with any of the above implementations, with reference to FIG. 6, the bendable tube 41 is provided with an aperture 418. The pulling wire 42 extends out of or into the bendable tube 41 via the aperture 418. The adjustable bendable sheath 4 further includes an elastic sealing member 44. The elastic sealing member 44 covers the outer peripheral wall of the pulling wire 42. The elastic sealing member 44 is arranged between the inner wall of the aperture 418 and the pulling wire 42; or the elastic sealing member 44 is arranged within the bendable tube 41 and seals the aperture 418.

Particularly, the aperture 418 may be the first opening 412, the second opening 413, and the third opening 414 in the first implementation, or any one of the first through hole 431 to the fourth through hole 434 in the second implementation.

Particularly, the elastic sealing member 44 may be connected between the inner wall of the aperture 418 and the peripheral wall of the pulling wire 42 in a sealing manner, or may be arranged within the inner cavity 417 of the bendable tube 41. The elastic sealing member 44 conforms to the outer lining layer 415 of the bendable tube 41 and covers the aperture 418.

Particularly, the elastic sealing member 44 is sealed between the pulling wire 42 and the bendable tube 41 to prevent blood from flowing into the bendable tube 41 via a gap between the bendable tube 41 and the pulling wire 42 when the adjustable bendable sheath 4 extends into the vessel.

Particularly, the elastic sealing member 44 is made of an elastic material, and particularly may be made of an elastic plastic, an elastic rubber, an elastic composite material, or the like. When the elastic sealing member 44 covers the pulling wire 42, the elastic sealing member 44 is in a compressed state. When the pulling wire 42 is being bent, the elastic sealing member 44 will deform with the pulling wire 42 to keep the pulling wire 42 covered. Therefore, when the pulling wire 42 is bent, the elastic sealing member 44 still remains sealed between the bendable tube 41 and the pulling wire 42.

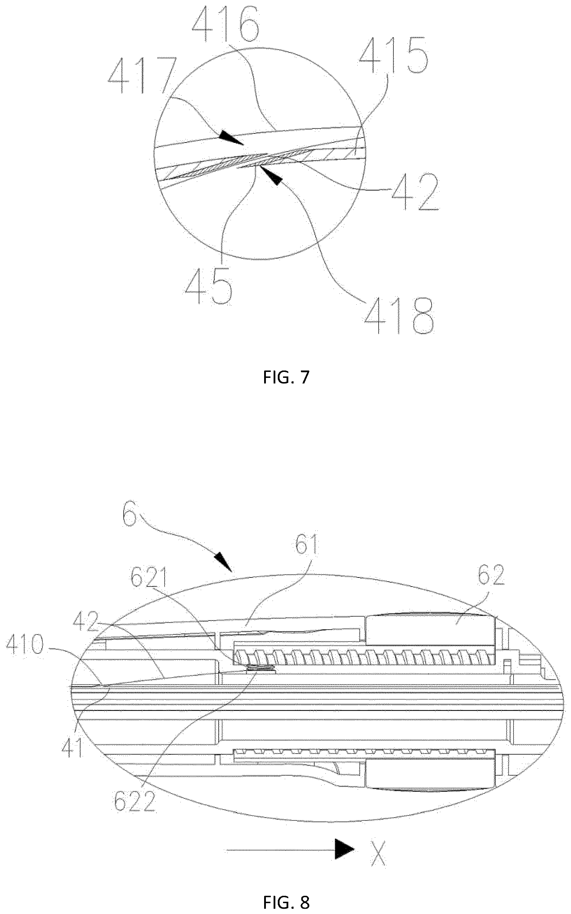

In combination with any of the above implementations, with reference to FIG. 7, the bendable tube 41 is provided with an aperture 418 and a reinforcing layer 45 arranged within the aperture 418. The pulling wire 42 extends out of or into the bendable tube 41 via the aperture 418. The reinforcing layer 45 surrounds the peripheral wall of the pulling wire 42. The reinforcing layer 45 is configured to increase the strength of the bendable tube 41at the aperture 418. Particularly, the reinforcing layer 45 may be made of a wear-resistant and tear-resistant plastic or rubber or polymer material or composite material. Furthermore, the reinforcing layer 45 may have a smooth surface to improve the smoothness between the pulling wire 42 and the bendable tube 41.

The aperture 418 may be the first opening 412, the second opening 413, or the third opening 414 in the first implementation, or may be any one of the first through hole 431 to the fourth through hole 434 in the second implementation.

By providing a reinforcing layer 45 at the aperture 418, which is capable of preventing the pulling wire 42 from tearing the bendable tube 41 in the process of driving the bendable tube 41 to be bent, the reliability and the service life of the adjustable bendable sheath 4 are improved.

It should be understood that a portion of the pulling wire 42, which is exposed outside the bendable tube 41, may be three or more sections, which will not be repeated herein.

With reference FIG. 1 to FIG. 3, the stent delivery device 100 includes the adjustable bendable sheath 4 according to any one of the above implementations and the sheath adjusting assembly 6 described. The sheath adjusting assembly 6 is connected to the other end of the pulling wire 42. The sheath adjusting assembly 6 is configured to pull the pulling wire 42 to drive the adjustable bendable sheath 4 to be bent.

By providing the sheath adjusting assembly to pull the pulling wire 42 to bend the adjustable bendable sheath 4. The bending degree of the adjustable bendable sheath 5 is adjusted by adjusting a distance the pulling wire 42 is pulled to move by the sheath adjusting assembly 6, so that the bending degree of the adjustable bendable sheath 4 follows that of the vessel, which facilitates the delivery of the stent 200 to the curved vessel by the stent delivery device 100.

In a possible implementation, with reference to FIG. 8, the sheath adjusting assembly 6 includes a fixed sleeve 61 and a rotary sleeve 62. The fixed sleeve 61 is fixedly mounted around the outer periphery of the adjustable bendable sheath 4. The rotary sleeve 62 is slidably mounted around the outer periphery of the adjustable bendable sheath 4. The fixed sleeve 61 and the rotary sleeve 62 are threadedly connected. The rotary sleeve 62 is connected to the other end of the pulling wire 42. When the rotary sleeve 62 is rotated, the rotary sleeve 62 moves away from the fixed sleeve 61, and the rotary sleeve 62 pulls the pulling wire 42 to bend the adjustable bendable sheath 4.

By providing the fixed sleeve 61 and the rotary sleeve 62 which are threadedly connected, the rotary sleeve 62 gradually pulls the pulling wire 42 to gradually bend the adjustable bendable sheath 4. When the adjustable bendable sheath 4 is bent, the adjustable bendable sheath 4 is capable of remaining in the bent state even if the rotary sleeve 62 stops rotating, which allows multiple adjustments in the operation to achieve the most appropriate bending degree of the adjustable bendable sheath 4.

Further, with reference to FIG. 8, an inner wall of the rotary sleeve 62 is spaced from the outer tube wall of the adjustable bendable sheath 4. A fixed block 621 and a pressing block 622 are provided on the inner wall of the rotary sleeve 62. The pulling wire 42 is connected to the fixed block 621. The pressing block 622 is configured to press the pulling wire 42 onto the fixed block 621.

Particularly, the fixed block 621 may be a bolt, and the pressing block 622 may be a nut. The distal end of the pulling wire 42 may be connected to the fixed block 621. The pressing block 622 is threadedly connected to the fixed block 621 so as to fix the pulling wire 42 to the fixed block 621.

In a possible implementation, with reference to FIG. 2 and FIG. 3, the stent delivery device 100 further includes an inner core 3 and a fixed assembly 1. The fixed assembly 1 is fixedly mounted around one end of the inner core 3. The fixed assembly 1 is configured to secure one end of the stent 200. The adjustable bendable sheath 4 is slidably mounted around the inner core 3. One end of the adjustable bendable sheath 4 abuts against the fixed assembly 1. The other end of the adjustable bendable sheath 4 is connected to the sheath adjusting assembly 6. An accommodating cavity 40 is defined between the adjustable bendable sheath 4 and the inner core 3 for accommodating the stent 200.

By providing the adjustable bendable sheath 4 and the inner core 3 to form the accommodating cavity 40, and due to the annular shape of the accommodating cavity 40, the tubular stent 200 may be tightened in a tubular form within the accommodating cavity 40. On the one hand, a space within the stent delivery device 100 is fully utilized. On the other hand, the stent 200 may be delivered after being tightened, which can reduce the profile of the stent delivery device 100, and thus reduce the interference of the stent delivery device 100 on the vessel. Moreover, the stent 200 is tubularly delivered, the stent 200 may easily return to its original shape after being released, to achieve treatment at the lesion site of the vessel.

Further, with reference to FIG. 2 and FIG. 3, one end of the adjustable bendable sheath 4 abuts against the fixed assembly 1. The adjustable bendable sheath 4 is capable of sliding relative to the inner core 3. When the adjustable bendable sheath 4 moves away from the fixed assembly 1, a portion of the stent 200, which exposes outside the adjustable bendable sheath 4, expands under its own tension.

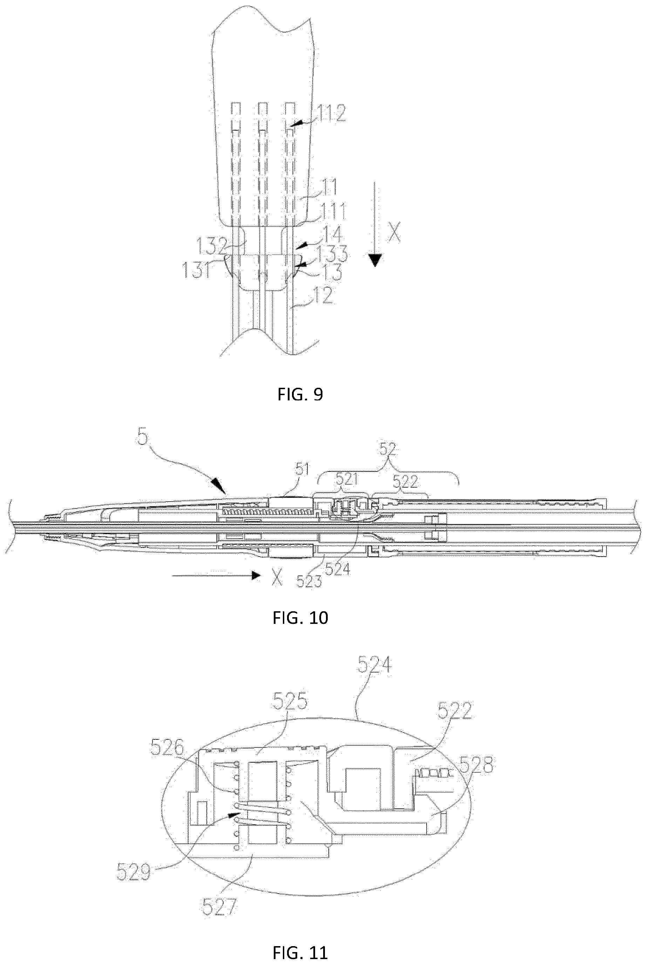

With reference to FIG. 3 and FIG. 9, the fixed assembly 1 includes a fixing member 11 and a limiting member 12. One end of the limiting member 12 extends out of the stent 200 (not shown) and is connected to the fixing member 11 so as to lock the stent 200. The limiting member 12 is capable of sliding relative to the fixing member 11. When the limiting member 12 moves away from the fixing member 11, the limiting member 12 releases the stent 200.

Particularly, with reference to FIG. 3 and FIG. 9, both the stent 200 and the limiting member 12 extend along the first direction X. The proximal end of the stent 200 is provided with a hollowing portion. One end of the limiting member 12 (a proximal end of the limiting member 12) may extend through the hollowing portion of the stent 200 and abuts against the fixing member 11, so that the proximal end of the stent 200 is tightened and fixed. The distal end of the stent 200 is adjacent to an operating end of the stent delivery device 100, and the distal end of the stent 200 is tightened and restrained by other position of the stent delivery device 100, such that the stent 200 is tightened and restrained to the stent delivery device 100, which brings the convenience for delivering the stent 200 into the vessel with the stent delivery device 100.

In a possible implementation, with reference to FIG. 3 and FIG. 9, the inner core 3 and the adjustable bendable sheath 4 are made of bendable materials, which may improve the bending performance of the stent delivery device 100, so that the stent delivery device 100 delivers the stent 200 to a curved vessel. Therefore, applications of the stent delivery device 100 are increased.

With reference to FIG. 3 and FIG. 9, the limiting member 12 extends within the accommodating cavity 40, and a gap between the limiting member 12 and the inner wall of the bendable tube 41 is used for receiving the stent 200.

With reference to FIG. 3 and FIG. 9, a plurality of limiting members 12 may be provided. When there is a plurality of limiting members 12, the plurality of limiting members 12 are annularly arranged within the accommodating cavity 40. The distal end of the stent 200 is mounted around the outer peripheries of the plurality of limiting members 12.

The limiting member 12 is accommodated between the inner core 3 and the adjustable bendable sheath 4, and the limiting member 12 is arranged within the stent 200, so that the limiting member 12 would not affect the release of the distal end of the stent 200.

With reference to FIG. 3 and FIG. 9, the limiting member 12 is made of a bendable material, which further increases the bending performance of the stent delivery device 100, so as to facilitate the delivery of the stent 200 to the curved vessel with the stent delivery device 100. applications of the stent delivery device 100 are increased.

Particularly, the limiting members 12 are separately arranged within the accommodating cavity 40. When the stent delivery device 100 enters into the curved vessel, the limiting elements 12 may bend with the inner core 3 and the adjustable bendable sheath 4, so that the stent delivery device 100 is capable of delivering the stent 200 into the curved vessel.

With reference to FIG. 3 and FIG. 9, the fixed assembly 1 further includes a guide member 13, which is arranged between the fixing member 11 and the sheath adjusting assembly 6. The guide member 13 is located adjacent to the fixing member 11. The limiting member 12 extends through and is slidably connected to the guide member 13. An accommodating space 14 is defined between the guide member 13 and the fixing member 11. The accommodating space 14 is configured to accommodate an end portion of the stent 200. The limiting member 12 passes through the end portion of the stent 200.

Particularly, one end of the limiting member 12 extends through the guide member 13 and into the accommodating space 14 so as to extend through the end portion of the stent 200 and abut against the fixing member 11.

Particularly, with reference to FIG. 9, the guide member 13 may be connected to the fixing member 11. The fixing member 11 is provided with a first surface 111 directly facing the guide member 13. The guide member 13 is provided with a second surface 131 directly facing the fixing member 11. A protrusion 132 may be provided on the second surface 131. The protrusion 132 may be connected to the first surface 111. A radial size of the protrusion 132 may be smaller than that of the first surface 111 and the second surface 131, that is, the protrusion 132 is sunken in the radial direction relative to the fixing member 11 and the guide member 13. The first surface 111, the second surface 131 and the outer peripheral surface of the protrusion 132 cooperatively define the accommodating space 14.

Further, the guide member 13 may be integrally formed with the fixing member 11 as one piece, that is, one end of the protrusion 132 is integrally formed with the guide member 13 and the other end of the protrusion 132 is integrally formed with the fixing member 11.

The fixing member 11 and the guide member 13 are provided in the fixing assembly 1, and the fixing member 11 works cooperatively with the limiting member 12 such that one end of the stent 200 is locked by the limiting member 12. The guide member 13 provides guiding for the limiting member 12, thus the limiting member 12 can move away from or close to the fixing member 11 in the first direction X. When the limiting member 12 moves away from the fixing member 11, one end of the stent 200 is disengaged from the limiting member 12 due to the blockage of the guide member 13, so that the stent 200 is released.

With reference to FIG. 9, the fixing member 11 is provided with a first hole 112. The guide member 13 is provided with a second hole 112. The second hole 133 is aligned with the first hole 112. The limiting member 12 extends through the second hole 112 and is aligned with or extends into the first hole 112.

Particularly, with reference to FIG. 9, the first surface 111 is provided with the first hole 112. Particularly, the first hole 112 may be a blind hole. The first hole 112 extends along the first direction X. One end of the limiting member 12 extends into or exits from the first hole 112 when being driven by an external force. By providing the first hole 112 in the fixing member 11, the limiting member 12 is capable of extending into the first hole 112, so as to limit one end of the limiting member 12 by the fixing member 11 to lock the stent 200, such that the stent 200 is not readily to be released.

Particularly, with reference to FIG. 9, the guide member 13 is provided with the second hole 133. The second hole 133 may extend through the guide member 13 along the first direction X. The second hole 133 is aligned with the first hole 112. The limiting member 12 extends through the second hole 133 and is slidably connected to the guide member 13.

Particularly, the proximal end of the limiting member 12 enters into the first hole 112 via the second hole 133. When the limiting member 12 moves, the limiting member 12 slides along the second hole 133.

By providing the second hole 133 in the guiding member 13, which serves to guide the limiting member 12, and the second hole 133 being aligned with the first hole 112, the limiting member 12 is capable of accurately extending into the first hole 112.

Particularly, when the limiting member 12 moves away from the fixing member 11 when an external force is applied, an end portion of the limiting member 12 is capable of retracting into the second hole 133, and the stent 200 is disengaged from the limiting member 12 due to the blockage of the guide member 13. The guide member 13 serves to facilitate the release of the stent 200 from the limiting member 12.

In a possible implementation, with reference to FIG. 9, a plurality of first holes 112 are provide. The plurality of first holes 112 are arranged along a circle. The second holes 133 have a number and an arrangement manner the same as those of the first holes 112. One first hole 112 is directly aligned with one second hole 133. A plurality of limiting member 12 is provided. The plurality of limiting members 12 are arranged in a tubular shape. One of the limiting members 12 extends through one of the second holes 133 and is aligned with or inserted into one of the first holes 112. Particularly, the stent 200 is of a tubular shape. The proximal end of the stent 200 is provided with a plurality of hollowing portions, and the plurality of limiting members 12 respectively extend through the plurality of hollow portions and abut against the fixing member 11, so that the proximal end of the stent 200 is tightened and fixed on the limiting member 12.

By providing the plurality of limiting members 12 to fix the stent 200 in a circumferential direction, the proximal end of the stent 200 is tightened and remains in a tubular shape. The firmness of the stent 200 fixed on the limiting member 12 may be improved.

In a possible implementation, with reference to FIG. 2 and FIG. 3, the stent delivery device 100 further includes a sheath sliding assembly 5, which is arranged between the fixed assembly 1 and the releasing assembly 2. The sheath sliding assembly 5 is mounted around the outer periphery of the adjustable bendable sheath 4. The sheath sliding assembly 5 is configured to drive the adjustable bendable sheath 4 to move away from the fixing member 11.

It should be understood that when the stent 200 is arranged within the vessel of a subject to be treated, the sheath sliding assembly 5 and the releasing assembly 2 are arranged outside the subject to be treated, so that the operator may control the expansion and release of the stent 200.

By operating the sheath sliding assembly 5 to control the adjustable bendable sheath 4 to move away from the fixing member 11, it is convenient for the operation and excellent in controllability.

In a possible implementation, with reference to FIG. 10, the sheath sliding assembly 5 includes a first sleeve 51 and a driving assembly 52 threadedly connected to the first sleeve 51. The first sleeve 51 is mounted around the outer periphery of the adjustable bendable sheath 4 and slidably relative to the adjustable bendable sheath 4. The driving assembly 52 is mounted around the outer periphery of the adjustable bendable sheath 4 and fixedly connected to the adjustable bendable sheath 4. When the driving assembly 52 rotates relative to the first sleeve 51, the driving assembly 52 gradually moves away from the first sleeve 51, and the adjustable bendable sheath 4 gradually moves away from the fixed assembly 11 under the action of the driving assembly 52 so that the proximal end of the stent 200 is gradually exposed from the adjustable bendable sheath 4.

It should be understood that the first sleeve 51 may serve as the sheath adjusting assembly 6.

In a possible implementation, with reference to FIG. 10, the driving assembly 52 includes a locking element 521 and a sliding sleeve 522. One end of the locking element 521 is threadedly connected to the first sleeve 51. The other end of the locking element 521 is snap-fit with the sliding sleeve 522. The locking element 521 is slidably connected to the adjustable bendable sheath 4. The sliding sleeve 522 is fixedly connected to the adjustable bendable sheath 4. When the locking element 521 is disengaged from the sliding sleeve 522, the sliding sleeve 522 is capable of driving the adjustable bendable sheath 4 to gradually move away from the fixing member 11.

By providing the locking element 521 and the sliding sleeve 522, with the locking element 521 being threadedly connected to the second sleeve 51, the adjustable bendable sheath 4 slowly retracts for a short distance by rotating the locking element 521 relative to the first sleeve 51, and the proximal end of the stent 200 gradually expands, while the locking element 521 is snap-fit with the sliding sleeve 522. When the locking element 521 is disengaged from the sliding sleeve 522, the sliding sleeve 522 is capable of pulling the adjustable bendable sheath 4 backwards for a longer distance after the restraint of the locking element 521 is removed, so that the adjustable bendable sheath 4 retracts rapidly for a longer course. Therefore, the proximal end and the distal end of the stent 200 expand.

Further, with reference to FIG. 10 to FIG. 11, the locking element 521 includes a second sleeve 523 and a switch 524 arranged on the second sleeve 523. The second sleeve 523 is mounted around the outer tube and slides relative to the outer tube. The switch 524 is slidably connected to the second sleeve 523 along a radial direction of the second sleeve 523. A buckling portion 528 of the switch 524 is snap-fit with the sliding sleeve 522. When the switch 524 slides to a pressed position, the buckling portion 528 of the switch 524 is disengaged from the sliding sleeve 522.

Further, the switch 524 is provided with a pressing portion 525, an elastic member 526 and a base 527 which are arranged along the radial direction of the second sleeve 523 in sequence. The pressing portion 525 is arranged on an outer peripheral surface of the second sleeve 523. The elastic member 526 elastically abuts between the pressing portion 525 and the base 527, so that a gap 529 is defined between the pressing portion 525 and the base 527. The buckling portion 528 extends from one end of the pressing portion 525 towards the sliding sleeve 522. When the pressing portion 525 abuts against the base 521, the switch 524 is positioned at the pressed position. When the sliding sleeve 522 abuts against the second sleeve 523 and the switch 524 rebounds from the pressed position under the action of the elastic member 526, the buckling portion 528 of the switch 524 is snap-fit with the sliding sleeve 522 under the action of the pressing portion 525.

In a possible implementation, with reference to FIG. 2 and FIG. 3, the stent delivery device 100 further includes a releasing assembly 2. The releasing assembly 2 is positioned on one side of the sheath adjusting assembly 6 which is away from the fixed assembly 1. The releasing assembly 2 is connected to the other end of the limiting member 12. The releasing assembly 2 is configured to drive the limiting member 12 to move away from or close to the fixing member 11.

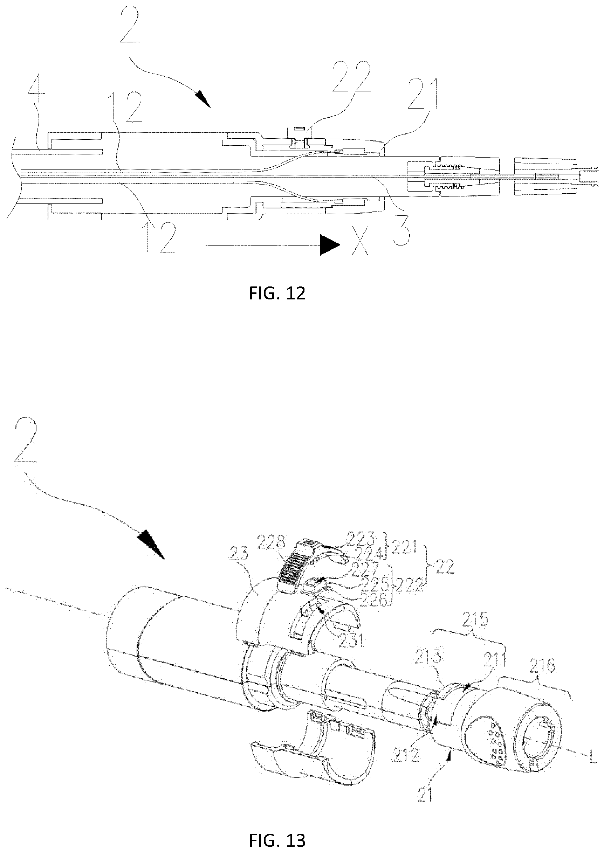

Particularly, with reference to FIG. 12, the releasing assembly 2 includes a movable member 21 and a locking member 22 connected to the movable member 21. The movable member 21 is connected to the other end of the limiting member 12 (the distal end of the limiting member 12). The locking member 22 is slidably connected to the movable member 21. When the locking member 22 is located at the first position, the movable member 21 is fixed relative to the fixing member 11 due to the restriction of the locking member 22. When the locking member 22 slides to the second position, the movable member 21 is capable of driving the limiting member 12 to move away the fixing member 11 so as to release the stent 200.

With reference to FIG. 12, The movable member 21 is configured to drive the limiting member 12 to move away from the fixing member 11 in the first direction X, so that the stent 200 is released by the limiting member 12. The locking member 22 is configured to lock the movable member 21, to prevent the movable member 21 from moving in the first direction X, and thus prevent the limiting member 12 from releasing the stent 200 due to unintentional touch and other reasons when there is no need to release the stent 200 which results in an incorrect position where the stent 200 is released and thus incapability of treating the vascular diseases.

Specifically, with reference to FIG. 12, the locking member 22 is slidably connected to the movable member 21. When the locking member 22 is located at a first position, the movable member 21 is fixed relative to the fixing member 11 under the restriction of the locking member 22. In other words, when the locking member 22 is in the first position, the movable member 21 is locked, so that the movable member 21 cannot move in the first direction X. At that time, the limiting member 12 abuts against the fixing member 11. When the locking member 22 slides to a second position, the movable member 21 can drive the limiting member 12 to move away from the fixing member 11 to release the stent 200. In other words, when the locking member 22 is located at the second position, the movable member 21 can move in the first direction X, when the limiting member 12 driven by the movable member 21 moves away from the fixing member, the limiting member 12 may be disengaged from the proximal end of the stent 200 when moving to a certain position, so that the stent 200 is released into the vessel.

The locking member 22 is provided to lock the movable member 21, so as to prevent the movable member 21 from driving the limiting member 12 to release the stent 200 when there is no need to release the stent 200. The locking member 22 fastens or releases the movable member 21 by moving to different positions, so as to control the movable member 21 to drive the limiting member 12 to release the stent 200 at an appropriate time. That is, the time and the position in which the stent 200 is released are controlled by changing the position of the locking member 22. Since the position of the locking member 22 is changeable, it may be rapidly determined that the movable member 21 is in a locked state or a released state, so that it is possible to perform the next step quickly, thereby saving the operation time.

In a possible embodiment, with reference to FIG. 13, an outer peripheral surface of the movable member 21 is provided with a first sliding groove 211 and a second sliding groove 212 communicating end to end. The first sliding groove 211 extends along a circumferential direction of the movable member 21. The second sliding groove 212 extends along the central axis L. One end of the locking member 22 can slide in the first sliding groove 211 and the second sliding groove 212.

Particularly, the movable member 21 may be of a cylindrical shape, with an axial direction thereof as the first direction X. Certainly, in other embodiments, the movable member 21 may also be of a square tubular shape or other tubular shape.

Due to the first sliding groove 211 and the second sliding groove 212 provided in the outer circumferential surface of the movable member 21, with the first sliding groove 211 extending along the circumferential direction of the movable member 21, and the second sliding groove 212 extending along the first direction X, when the locking member 22 is located in the first sliding groove 211, the locking member 22 locks the movable member 21 in the first direction X to prevent the movable member 21 from moving in the first direction X, and the limiting member 12 abuts against the fixing member 11. If the limiting member 12 extends through the stent 200, the limiting member 12 fixes the stent 200 on the stent delivery device 100. When the locking member 22 slides to the second sliding groove 212, the locking member 22 releases the movable member 21, so that the movable member 21 is capable of moving in the first direction X, and in turn the movable member 21 is capable of driving the limiting member 12 to move away from the fixing member 11 in the first direction X, so as to release the stent 200 on the limiting member 12, or the movable member 21 is capable of driving the limiting member 12 to approach to the fixing member 11 along the first direction X, so as to make the limiting member 12 return to an initial position after the stent 200 is released.

Further, the first position and the second position are located at the first sliding groove 211 and the second sliding groove 212, respectively. Particularly, the first position may be any position in the first sliding groove 211, and the second position may be any position in the second sliding groove 212. That is, when the locking member 22 is located in the first sliding groove 211, the movable member 21 is fixed relative to the fixing member 11 under the restriction of the locking member 22. When the locking member 22 slides to the second sliding groove 212, the movable member 21 is capable of driving the limiting member 12 to move away from the fixing member 11 so as to release the stent 200.

Further, with reference to FIG. 13, the movable member 21 has a first end surface 213, through which the second sliding groove 212 extends. When the movable member 21 moves away from the fixing member 11, the locking member 22 is capable of sliding out of the second sliding groove 212 until it is disengaged from the movable member 21. It should be understood that the first end surface 213 is adjacent to the fixing member 11.

Due to the second sliding groove 212 extending through the first end surface 213, the locking member 22 is capable of sliding out of the second sliding groove 212, and the movable member 21 is then disengaged from the locking member 22. The movable member 21 is controlled to drive the limiting member 12 along the first direction X, so that the limiting member 12 may be quickly separated from the fixing member 11 so as to quickly release the stent 200. Therefore, the stent 200 is released at an accurate position and the surgical time is reduced. Moreover, the movable member 21 disengaged from the locking member 22 is capable of moving freely along an appropriate course, to drive the limiting member 12 to move an appropriate distance, such that the limiting member 12 is capable of releasing the stent 200 without moving too far from the fixing member 11 which results in difficulty of returning to the initial position.

With reference to FIG. 13, the releasing assembly 22 further includes a third sleeve 23. The third sleeve 23 is mounted around an outer periphery of the movable member 21. The third sleeve 23 is provided with a limiting hole 231. The locking member 22 is arranged on the third sleeve 23. The locking member 22 is slidably connected to the movable member 21 through the limiting hole 231.

It should be understood that the central axis L of the third sleeve 23 runs along the first direction X. The third sleeve 23 can restrict the locking member 22 in a radial direction, so that the locking member 22 may be slidably connected to the movable member 21.

In a possible embodiment, the limiting hole 231 is an elongated hole. Particularly, the limiting hole 231 may extend along a circumferential direction of the third sleeve 23. When the locking member 22 slides, the third sleeve 23 is fixed relative to the fixing member 11, and the locking member 22 can slide in the limiting hole 231. In other embodiments, the locking member 22 may be fixed on the third sleeve 23. When the locking member 22 slides, the locking member 22 and the third sleeve 23 rotate together around the first direction X, to allow one end of the locking member 22 to be slidably connected to the movable member 21.

Further, with reference to FIG. 13, the limiting hole 231 extends along the circumferential direction of the third sleeve 23. The locking member 22 can slide from one end of the first sliding groove 211 to the other end of the first sliding groove 211 along the limiting hole 231. An inner wall of the limiting hole 231 restricts the locking member 22 to be fixed relative to the third sleeve 23 in the axial direction.

The limiting hole 231 which is configured as an elongated hole extending in the circumferential direction provides a sliding space for the locking member 22, so that the locking member 22 is capable of sliding along the circumferential direction of the third sleeve 23 with the locking member 22 being restricted to be axially fixed relative to the third sleeve 23. Therefore, the position of the locking member 22 is changeable, which facilitates quick recognition of the movable member 21 in the locked state or the released state, and thus increase the speed of releasing the stent 200.

Further, when the locking member 22 slides out of the second sliding groove 212, the movable member 21 is disengaged from the third sleeve 23 and the locking member 22, and the limiting member 12 moves away from the fixing member 11 when being driven by the movable member 21, and then releases the stent 200 to achieve the treatment of the vascular disease.

In a possible embodiment, with reference to FIG. 13, the movable member 21 includes a connecting portion 215 and an operating portion 216 connected to each other. The connecting portion 215 is provided with the first sliding groove 211 and the second sliding groove 212. The third sleeve 23 is mounted around an outer peripheral surface of the connecting portion 215. The connecting portion 215 is connected to the limiting member 12. The operating portion 216 is arranged apart from the third sleeve 23, and is configured to move under an external force, and drive the connecting portion 215 and the limiting member 12 to move away from or close to the fixing member 11.

Particularly, with reference to FIG. 13, the operating portion 216 may be a handheld structure. When the movable member 21 is released from the locking member 22, an operator holds the operating portion 216 with his or her hand and pulls the operating portion 216 towards a distal end of the stent delivery device 100. The operating portion 216 drives the connecting portion 215 to approach to the distal end of the stent delivery device 100, and thus the limiting member 12 approaches to the distal end of the stent delivery device 100 under the action of the connecting portion 215, so that the limiting member 12 gradually moves away from the fixing member 11, and thus the stent 200 is quickly released at the lesion site of the vessel.

In a possible embodiment, with reference to FIG. 13, the locking member 22 includes a toggle portion 221 and a sliding portion 222 connected to each other. The toggle portion 221 abuts against an outer peripheral surface of the third sleeve 23. The sliding portion 222 passes through the limiting hole 231 and abuts against an inner peripheral surface of the third sleeve 23. The toggle portion 221 is configured to slide relative to the third sleeve 23 under an external force, and drive the sliding portion 222 to slide in the first sliding groove 211 and the second sliding groove 212 through the limiting hole 231 of the third sleeve 23.

The sliding portion 222 may slide along the limiting hole 231 by sliding the toggle portion 221 by the operator, that is, the operator may make the locking member 22 lock or release the movable member 21 by operating the toggle portion 221. The operation is simple and convenient, with high feasibility, and quick implementation, and the efficiency of releasing the stent 200 is improved.

Further, the toggle portion 221 is detachably connected to the sliding portion 222, so that the locking member 22 may be conveniently and quickly mounted on the third sleeve 23.

In a possible embodiment, with reference to FIG. 13, the toggle portion 221 is snap-fit with the sliding portion 222. The toggle portion 221 includes a cover plate 223 and a hook 224 arranged on the cover plate 223. The cover plate 223 abuts against the outer surface of the third sleeve 23. The sliding portion 222 is provided with a first sliding portion 225 and a second sliding portion 226 connected to each other, with a through hole 227 extending through the first sliding portion 225 and the second sliding portion 226. The first sliding portion 225 is slidably engaged in the limiting hole 231 of the third sleeve 23, and the second sliding portion 226 is slidably engaged in the first sliding groove 211 and the second sliding groove 212 of the movable member 21. Particularly, the first sliding portion 225 is configured to slide along the limiting hole 231 of the third sleeve 23 when being driven by the toggle portion 221. In one embodiment, the first sliding portion 225 is configured to be at least partially accommodated in the limiting hole 231 of the third sleeve 23. The second sliding portion 226 is configured to slide in the first sliding groove 211 and the second sliding groove 211 of the movable member 21. The hook 224 is engaged with the second sliding portion 226 through the through hole 227, so that one end of the first sliding portion 225 abuts against the cover plate 223.

In other embodiments, the toggle portion 221 may be threadedly or magnetically connected to the sliding portion 222.

Further, with reference to FIG. 13, textures 228 are provided on an outer surface of the cover plate 223, such that when the operator manually toggles the cover plate 223, the textures 228 serves to avoid skidding.

In combination with any one of the foregoing implementations, with reference to FIG. 1 and FIG. 2, the sheath adjusting assembly 6, the sheath sliding assembly 5 and the releasing assembly 2 form a handle portion of the stent delivery device 100.

The stent delivery device 100 according to the present application can be operated with the following process.

During the operation, firstly, a vessel of a subject to be treated is punctured, a guide wire is inserted, and then the stent delivery device 100 is delivered into the aorta over the guide wire. Under X-ray fluoroscopy monitoring, the stent delivery device 100 is advanced to the vicinity of a lesion site of the vessel. The rotary sleeve 62 is rotated. The rotary sleeve 62 drives the pulling wire 42 to move, so that the bendable tube body 41 is bent. The bendable tube body 41 is bent for achieving an appropriate angle by adjusting the turn of rotations of the rotary sleeve 62. Then, the bendable tube body 41 is advanced into the bended vessel., the locking element 521 is rotated, and the adjustable bendable sheath 4 is slowly retracted, so that the proximal end of the stent 200 slowly expands. When the stent 200 is released at a certain position, the stent 200 does not fully expand yet since the proximal end of the stent 200 is still restrained by the limiting member 12. The stent 200 is moved to the most appropriate releasing position by moving the stent delivery device. The switch 524 on the locking element 521 is pressed, and in the meanwhile the sliding sleeve 522 is pulled backwards. A main body of the stent 200 expands completely. The locking member 22 is toggled to the second sliding groove 212, and the movable member 21 is pulled backwards to make the limiting member 12 move away from the fixing member 11 and further release the proximal end of the stent 200. The stent 200 is completely released from the stent delivery device 100, and the releasing process is completed. At the end, the stent delivery device 100 is withdrawn from the subject to be treated along the guide wire.

The above are a part of the embodiments of the present application. It should be noted that for those ordinarily skilled in the art, several improvements and modifications may be made without departing from the principle of the present application, and these improvements and modifications are regarded to be within the protective scope of the present application.

## Claims

1. An adjustable bendable sheath for delivering a stent into a vessel, comprising:
a bendable tube; and
a pulling wire, wherein one end of the pulling wire is fixedly connected to one end of the bendable tube, an other end of the pulling wire extends out of the bendable tube and then into the bendable, and at the last extends out an other end of the bendable tube, and wherein when the other end of the pulling wire is pulled, the one end of the pulling wire drives the one end of the bendable tube to approach to the other end of the pulling wire, so that the bendable tube is bent.

2. The adjustable bendable sheath according to claim 1, wherein an outer tube wall of the bendable tube is provided with a fixed portion and a first opening, the one end of the pulling wire is fixed to the fixed portion, and the other end of the pulling wire extends into the bendable tube via the first opening.

3. The adjustable bendable sheath according to claim 2, wherein the outer tube wall of the bendable tube is further provided with a second opening, the first opening is positioned between the second opening and the fixed portion, and the other end of the pulling wire extends out of the outer tube wall of the bendable tube via the second opening.

4. The adjustable bendable sheath according to claim 3, wherein the first opening and the second opening are arranged along an axial direction of the bendable tube.

5. The adjustable bendable sheath according to claim 3, wherein the outer tube wall of the bendable tube is further provided with a third opening, the second opening is positioned between the first opening and the third opening, and the other end of the pulling wire extends into the bendable tube via the third opening.

6. The adjustable bendable sheath according to claim 5, wherein the first opening, the second opening and the third opening are collinear along the axial direction of the bendable tube.

7. The adjustable bendable sheath according to claim 6, wherein the outer tube wall of the bendable tube is further provided with a fourth opening, the fourth opening is adjacent to the other end of the bendable tube, and the other end of the pulling wire extends out of the bendable tube via the fourth opening.

8. The adjustable bendable sheath according to claim 6, wherein an end surface of the other end of the bendable tube is provided with a fourth opening, and the other end of the pulling wire extends out of the bendable tube via the fourth opening.

9. The adjustable bendable sheath according to claim 7, wherein the bendable tube is provided with an inner lining layer and an outer lining layer mounted around an outer periphery of the inner lining layer, with a gap provided between the outer lining layer and the inner lining layer to form an inner cavity, the first opening, the second opening, the third opening and the fourth opening communicate with the inner cavity, and the pulling wire is partially arranged in the inner cavity.

10. The adjustable bendable sheath according to claim 1, wherein a fixed portion is arranged within the bendable tube, the one end of the pulling wire is fixed to the fixed portion, the outer tube wall of the bendable tube is provided with a first through hole and a second through hole, the first through hole is provided between the second through hole and the fixed portion, and the pulling wire extends out of the bendable tube via the first through hole and into the bendable tube via the second through hole.

11. The adjustable bendable sheath according to claim 10, wherein the outer tube wall of the bendable tube is further provided with a third through hole, a fourth through hole and a fifth through hole, the first through hole, the second through hole, the third through hole, the fourth through hole and the fifth through hole are collinear along an axial direction of the bendable tube, and the pulling wire extends out of the bendable tube via the third through hole and into the bendable tube via the fourth through hole until the pulling wire extends out of the bendable tube via the fifth through hole.

12. The adjustable bendable sheath according to any one of claims 1 to 11, wherein the bendable tube is provided with an aperture, the pulling wire extends out of or into the bendable tube via the aperture, the adjustable bendable sheath further comprises an elastic sealing member, the elastic sealing member covers an outer peripheral wall of the pulling wire, and the elastic sealing member is arranged within the bendable tube and seals the aperture.

13. The adjustable bendable sheath according to any one of claims 1 to 11, wherein the bendable tube is provided with an aperture and a reinforcing layer arranged within the aperture, the pulling wire extends out of or into the bendable tube via the aperture, the reinforcing layer surrounds an outer peripheral wall of the pulling wire, and the reinforcing layer is configured to enhance the strength of the bendable tube at the aperture.

14. A stent delivery device for releasing a stent into a vessel, comprising the adjustable bendable sheath according to any one of claims 1 to 13, and further comprising a sheath adjusting assembly, wherein the sheath adjusting assembly is connected to the other end of the pulling wire, and the sheath adjusting assembly is configured to pull the pulling wire.

15. The stent delivery device according to claim 14, wherein the sheath adjusting assembly comprises a fixed sleeve and a rotary sleeve, the fixed sleeve is fixedly mounted around an outer periphery of the adjustable bendable sheath, the rotary sleeve is slidably mounted around an outer periphery of the adjustable bendable sheath, the fixed sleeve and the rotary sleeve are threadedly connected, the rotary sleeve is connected to the other end of the pulling wire, and wherein when the rotary sleeve is rotated, the rotary sleeve moves away from the fixed sleeve, and the rotary sleeve pulls the pulling wire to bend the adjustable bendable sheath.

16. The stent delivery device according to claim 15, wherein an inner wall of the rotary sleeve is spaced from the outer tube wall of the adjustable bendable sheath, a fixed block and a pressing block are provided on the inner wall of the rotary sleeve, the pulling wire is connected to the fixed block, and the pressing block is configured to press the pulling wire on the fixed block.

17. The stent delivery device according to claim 15, wherein the stent delivery device further comprises an inner core and a fixed assembly, the fixed assembly is fixedly mounted around one end of the inner core, the fixed assembly is configured to fix one end of the stent, the adjustable bendable sheath is slidably mounted around the inner core, one end of the adjustable bendable sheath abuts against the fixed assembly, an other end of the bendable sheath is connected to the sheath adjusting assembly, an accommodating cavity is defined between the adjustable bendable sheath and the inner core, and the accommodating cavity is configured to accommodate the stent.

18. The stent delivery device according to claim 17, wherein the fixed assembly comprises a fixing member and a limiting member, one end of the limiting member extends out of the stent and abuts against the fixing member to lock the stent, the limiting member is slidably connected to the fixing member, and wherein when the limiting member moves away from the fixing member, the limiting member releases the stent.

19. The stent delivery device according to claim 18, wherein the limiting member extends within the accommodating cavity, and a gap between the limiting member and an inner wall of the bendable tube is used for receiving the stent.

20. The stent delivery device according to claim 18, wherein the limiting member is made of a bendable material.

21. The stent delivery device according to claim 19, wherein the fixed assembly further comprises a guide member, the guide member is arranged between the fixing member and the sheath adjusting assembly, the limiting member extends through and is slidably connected to the guide member, an accommodating space is defined between the guide member and the fixing member, the accommodating space is configured to accommodate an end portion of the stent, and the limiting member extends through the end portion of the stent.

22. The stent delivery device according to claim 21, wherein the fixing member is provided with at least one first hole, the guide member is provided with at least one second hole, the second hole is aligned with the first hole, and the limiting member extends through the second hole and is aligned with or enters into the first hole.

23. The stent delivery device according to claim 22, wherein a plurality of limiting members is provided, the at least one first via hole comprises a plurality of first via holes, and the plurality of first via holes are arranged along a circle, and a number and an arrangement of the at least one second via hole are the same as those of the first via holes.

24. The stent delivery device according to claim 17, wherein the stent delivery device further comprises a sheath sliding assembly, the sheath sliding assembly is fixedly mounted around the adjustable bendable sheath, the sheath sliding assembly is connected to the sheath adjusting assembly, and the sheath sliding assembly is configured to drive the adjustable bendable sheath to slide relative to the inner core, so that the stent exposes from the adjustable bendable sheath.

25. The stent delivery device according to claim 24, wherein the sheath sliding assembly comprises a first sleeve and a driving assembly threadedly connected to the first sleeve, the first sleeve is slidably mounted around an outer periphery of the adjustable bendable sheath, the driving assembly is fixedly mounted around the outer periphery of the adjustable bendable sheath, and wherein when the driving assembly rotates relative to the first sleeve, the driving assembly gradually moves away from the first sleeve, and the adjustable bendable sheath gradually moves away from the fixed assembly under the action of the driving assembly.

26. The stent delivery device according to claim 25, wherein the driving assembly comprises a locking element and a sliding sleeve, one end of the locking element is threadedly connected to the first sleeve, and the other end of the locking element is detachably connected to the sliding sleeve; the locking element is slidably connected to the adjustable bendable sheath, the sliding sleeve is fixedly connected to the adjustable bendable sheath; and wherein when the locking element is disengaged from the sliding sleeve, the sliding sleeve is capable of driving the adjustable bendable sheath to gradually move away from the fixed assembly.

27. The stent delivery device according to claim 24, wherein the stent delivery device further comprises a releasing assembly, wherein the sheath adjusting assembly is positioned between the releasing assembly and the fixed assembly, the releasing assembly is connected to an other end of the limiting member, and the releasing assembly is configured to drive the limiting member to move away from or close to the fixing member.

28. The stent delivery device according to claim 27, wherein the releasing assembly comprises a movable member and a locking member connected to the movable member, the movable member is connected to an other end of the limiting member, the locking member is slidably connected to the movable member; when the locking member is in a first position, the movable member is fixed relative to the fixing member under the restriction of the locking member; and wherein when the locking member slides to a second position, the movable member is capable of driving the limiting member to move away from the fixing member so as to release the stent.

29. The stent delivery device according to claim 28, wherein an outer peripheral surface of the movable member is provided with a first sliding groove and a second sliding groove communicating end to end, the first sliding groove extends along a circumferential direction of the movable member, the second sliding groove extends along an axial direction of the adjustable bendable sheath, one end of the locking member is capable of sliding in the first sliding groove and the second sliding groove, and the first position and the second position are in the first sliding groove and the second sliding groove, respectively.

30. The stent delivery device according to claim 29, wherein the movable member is provided with a first end surface, the second sliding groove extends through the first end surface, and wherein when the movable member moves away from the fixing member, the locking member is capable of sliding out via the second sliding groove until the locking member is disengaged from the movable member.

31. The stent delivery device according to claim 30, wherein the releasing assembly further comprises a first sleeve, the first sleeve is mounted around an outer periphery of the movable member, the first sleeve is provided with a limiting hole, the limiting hole extends along a circumferential direction of the first sleeve, the locking member is arranged on the first sleeve, the locking member is capable of sliding from one end of the first sliding groove to an other end of the first sliding groove along the limiting hole, and an inner wall of the limiting hole limits the locking member from being fixed relative to the first sleeve in an axial direction.

32. The stent delivery device according to claim 27, wherein the sheath adjusting assembly, the sheath sliding assembly, and the releasing assembly form a handle portion of the stent delivery device.
